# EUROPEAN PATENT SPECIFICATION

(11) **EP 0 941 361 B1**
(45) Date of publication and mention of the grant of the patent: **28.11.2001**
(21) Application number: 97910556.6
(22) Date of filing: 31.10.1997
(51) Int. Cl.: C12P 33/02, C12P 33/08, C12P 33/12, C07J 5/00

(54) **A METHOD FOR THE PREPARATION OF STEROID COMPOUNDS**
VERFAHREN ZUR HERSTELLUNG VON STEROIDVERBINDUNGEN
PROCEDE DE PREPARATION DE COMPOSES STEROIDES

(30) Priority: 02.11.1996 GB 9622884
(43) Date of publication of application: 15.09.1999
(73) Proprietor: Duramed Europe Limited, Oxford OX4 4GA (GB)
(72) Inventor: ROBERTS, Stanley, Michael, Devon EX9 7AB (GB); EDDOLLS, Jonathan, Paul, Bristol BS16 6SG (GB); WILLETTS, Andrew, John, Devon EX5 5ER (GB); ATKINSON, Anthony, Salisbury, Wiltshire SP4 6JJ (GB); MURPHY, Jonathan, Paul, Oxfordshire OX11 9JS (GB)
(74) Representative: Lipscombe, Martin John
(86) International application number: GB9703003
(87) International publication number: WO9820151

(56) References cited:
- EP-A- 0 042 451
- EP-A- 0 072 546
- EP-A- 0 095 894
- EP-A- 0 322 630
- CH-A- 399 453
- CHEMICAL ABSTRACTS, vol. 82, no. 25, 23 June 1975 Columbus, Ohio, US; abstract no. 167342, HOLLAND HERBERT AND AL.: "Mechanism of the microbial hydroxylation of steroids. 1. C-21 hydroxylation of progesterone by Aspergillus niger ATCC 9142" page 254; XP002056381 & CAN.J.CHEM, vol. 53, no. 6, 1975, pages 845-854, cited in the application

## Description

### Field of the Invention

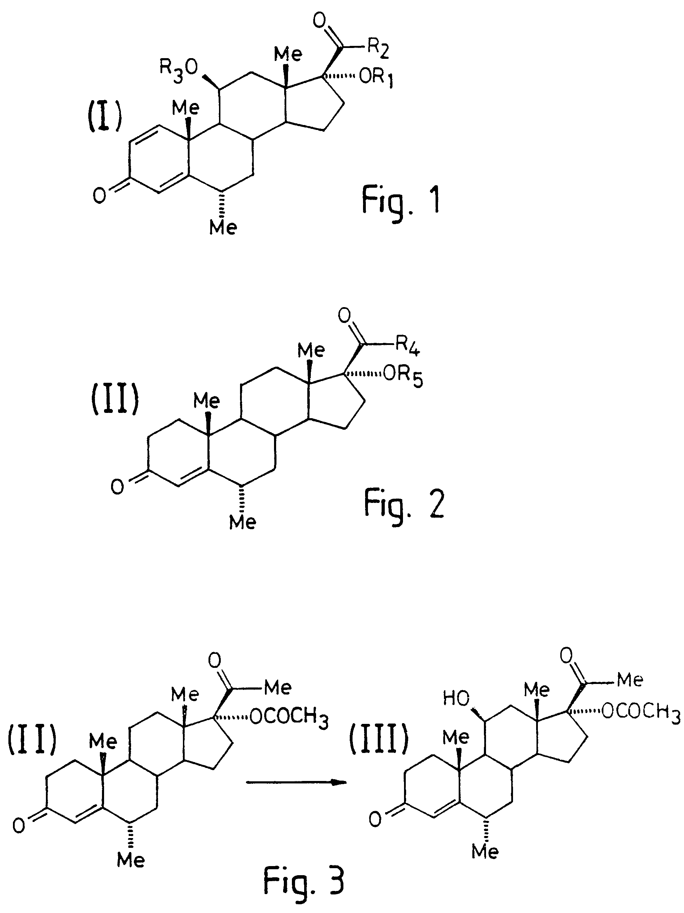

The present invention relates to a method for the preparation of adrenocorticoid steroid compounds from a suitable precursor compound. In particular the invention relates to the production of methylprednisolone and its derivatives having a general formula (I) shown below in Figure 1.

### Background of the Invention

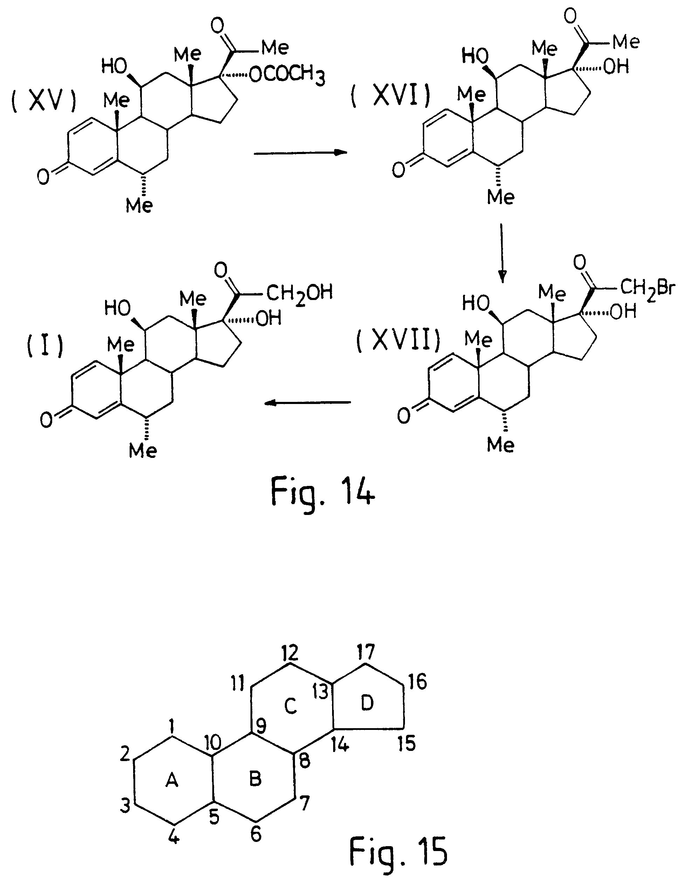

Steroids are a family of compounds that contain the four-membered perhydro-cyclopentanophenanthrene ring system. The four rings are named A, B, C and D and are shown in Figure 15. In steroids, a side chain of varying length is present on the D ring.
Figure 15 also shows the convention which has been adopted for numbering of the carbon atoms in steroids. Substituents of the steroid ring system are termed "α" or "β" if they lie respectively below or above the plane of the ring. Conventionally, α components are shown with a dotted bond to the rings, and β components with a solid bond.

Steroids are widely distributed in plants and animals and are among the most important natural products, having first been isolated and studied in the early nineteenth century. Steroids include: sterols, bile acids, sex hormones, the steroid hormones of the adrenal cortex ("adrenocorticoid steroids"), and the cardiac aglycones.

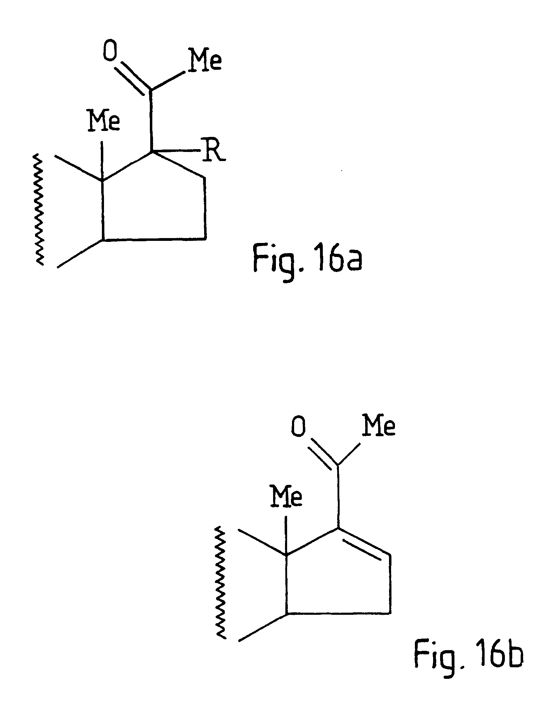

Sex hormones include the estrogens, androgens, and progestogens. Progestogens are steroids wherein the D ring has the structure shown in Figure 16a or 16b. where R = H, OH, O-acetyl etc. Particular examples of progestogens are the compounds medroxyprogesterone and medroxyprogesterone acetate ("MPA"), which conform to the general formula (II) shown in Figure 2, when R₄ = CH₃, and when R₅ = H (for medroxyprogesterone) or R₅ = COCH₃ (for MPA).

The steroid methylprednisolone has the chemical name 11β, 17α, 21-trihydroxy-6α-methyl, pregn-1,4-diene-3,20-dione and is within the scope of general formula (I, Figure 1), where (for methylprednisolone) R₁ and R₃ = H and R₂ = CH₂OH. Methylprednisolone is a potent anti-inflammatory adrenocorticoid steroid and is widely used in the preparation of anti-inflammatory medicaments. Such medicaments may be for intramuscular, intrasynovial, soft tissue, intravenous or intralesional action for the treatment of a variety of disorders for example endocrine disorders, rheumatic disorders, respiratory diseases and gastrointestinal diseases amongst others.

Currently, methylprednisolone is manufactured by complex chemical processes which are time consuming and expensive. Where microbiological processes have been used in the prior art, problems often encountered include the reaction products being obtained at an unsatisfactory level of purity, or at an unsatisfactory yield level, or a combination of both. Consequently, further purification steps are required before the compound can be used for the preparation of a pharmaceutically acceptable product, and/or larger quantities of starting material are required in order to obtain a sufficiently high yield of end product.
Additional purification steps or the use of larger quantities of starting material make the end product expensive for the consumer.

Other strategies used previously include the synthesis of specifically designed intermediates which can be easily converted to the desired end product. However, the problem with this approach is that such intermediates are often complex and uneconomic to produce in bulk.

A further problem encountered in the prior art methods for the production of methylprednisolone is that the level of diastereomeric purity of the compound is often unsatisfactory, particularly at the carbon-11 position of the steroid molecule, where either 11β or 11α hydroxylation may occur. Consequently further processing is required to separate the two enantiomers making the process lengthy and expensive. Also, such further processing tends to decrease the yield of the end product.

A variety of starting materials have been used in the prior art methods for the preparation of methylprednisolone. However, these starting materials have the problem that they either require complex processing, or are too expensive to purchase in bulk, or have to be specifically synthesised, or are not pharmaceutically acceptable at the outset, or a combination of such factors.

The chemical steps used in the prior art, particularly for the dehydrogenation of the "A"-ring of a steroid precursor, often require the use of highly toxic materials such as selenium dioxide. In large scale production, use of such toxic materials is particularly hazardous to the health of production staff. Alternative selenium derivatives have been sought to reduce these hazards, but often with unsatisfactory results.

A further problem is that the hydroxylation at the carbon-21 position on the "D"-ring of a progestogen steroid having a carbon-20 ketone group, and a protected or unprotected hydroxy group at the carbon-17 position, is generally difficult as the reaction tends to cause rearrangements of the "D"-ring.

An object of the present invention is to provide a novel method for the production of compounds having the general formula (I), whilst seeking to alleviate the above mentioned problems, (conveniently by utilising a combination of microbiological, enzymatic and chemical means) by converting a suitable steroid starting material which is readily available, cheap to purchase in bulk and physiologically acceptable at the outset.

A further object of the present invention is to provide a high yield and high purity end product, which can be produced more economically.

A still further object of the present invention is to provide an alternative, less toxic material for use in the chemical Δ¹⁻² dehydrogenation step in the "A"-ring of a steroid molecule.

### Summary of the Invention

According to the present invention there is provided a method for the production of an adrenocorticoid steroid represented by the general formula (I) shown in Figure 1, wherein R₁ may be H, alkyl, substituted alkyl, silyl, acyl, acyloxy or carbamoyl, R₂ may be methyl, mono- or disubstituted methyl, substituted alkyl, methyl or substituted alkyl hydroxy, silyoxy, alkyloxy, acyl, acyloxy or carbamoyl, and R₃ is H; comprising treatment of a precursor thereof so as to effect the following: hydroxylation at the 11β position; formation of a double bond between positions 1 and 2; and hydroxylation at position 21.

Preferably the steroid produced is one where R₁=H or COCH₃ and R₂=CH₂OH.

Those skilled in the art will appreciate that formation of the double bond between positions 1 and 2 is brought about by dehydrogenation (Δ¹⁻² dehydrogenation), wherein the term refers typically to the loss of hydrogen, but also encompasses the loss of other atoms or groups from position 1 or 2.

The steps in accordance with the method of the invention may be performed by microbial biotransformation, enzymatic or chemical means, or any combination thereof. Conveniently, the 11β hydroxylation and Δ¹⁻² dehydrogenation are both achieved by microbial biotransformation, and the hydroxylation at position 21 is typically achieved by chemical means, although in one particular embodiment all three steps are achieved by microbial biotransformation.

The precursor is a steroid conforming to the general formula (II) shown in Figure 2, wherein R₅ may be H, alkyl, substituted alkyl, silyl, acyl, acyloxy, carbamoyl, and R₄ may be methyl, mono- or disubstituted methyl, substituted alkyl, methyl or substituted alkyl hydroxy, silyloxy, alkyloxy, acyl, acyloxy, carbamoyl. Most preferably, R₄ = CH₃ and R₅ = H or COCH₃. This compound (medroxyprogesterone, where R₅ = H; medroxyprogesterone acetate ["MPA"] where R₅ = COCH₃) is readily available at comparatively low cost.

It will be appreciated that in synthesising methylprednisolone (or a derivative thereof) from medroxyprogesterone or MPA or a derivative thereof, each of the steps in the method defined above must be performed. Where the steps are performed substantially simultaneously, each step may be considered as being performed on a precursor having the general formula (II). However, the steps may also be performed sequentially, such that only the initial step of the method is performed on a compound according to general formula (II). Accordingly it will be understood that the term "precursor" makes reference not only to compounds according to general formula (II) and other related, suitable compounds, but also to intermediates prepared by subjecting compounds according to general formula (II) to treatment of any one, or any two, of the following: hydroxylation at the 11β position; formation of a double bond between positions 1 and 2; and hydroxylation at position 21.

In a preferred embodiment the C21 hydroxylation step is performed on a precursor compound which has an -OR group attached to carbon 17 (where R may be H, acetyl, akyl etc). Such a reaction has proved generally difficult to perform without causing rearrangements of the D ring.

In one method, the 11β-hydroxylation of the precursor may be performed by a system comprising a hydroxylase enzyme which effects 11β-hydroxylation. An example of such an enzyme is that produced by members of the genus *Curvularia*, especially *C*. *lunata*. Appropriate microbial biotransformation methods are discussed by Shull & Kita (1955 J. Amer. Chem. Soc. 77, 763).

The Δ¹⁻² dehydrogenation of the precursor may be performed by a system comprising a dehydrogenase enzyme which effects Δ¹⁻² dehydrogenation. An example of such an enzyme is that produced by members of the genus *Nocardia*, especially *N*. *simplex*. Appropriate microbial biotransformation methods are described by Nobile *et al*., (1955 J. Amer. Chem. Soc. 77, 4184).

The enzyme systems described above may comprise cells of the organisms producing the desired enzymes *in situ*, or may comprise a cell-free extract comprising the relevant enzyme, or may comprise a purified enzyme preparation. It should be mentioned that the enzymes involved have not been well characterised and that, in practice, the microbial transformations may involve the action of two or more different enzymes. The term "enzyme system" should be construed accordingly, and is intended to refer to a reaction system comprising the enzyme substrate(s) and appropriate enzyme(s) in conditions which allow for the relevant enzymatic reactions to occur.

In another embodiment, the Δ¹⁻² dehydrogenation of the precursor is performed by chemical means and conveniently comprises a catalytic step using, for example, diphenyl diselenide.

In one embodiment, the hydroxylation at the carbon-21 position of the precursor is performed by chemical means and comprises the steps of bromination followed by elimination and saponification. Suitable techniques for accomplishing this step are described in the prior art, for example, by Elks *et al*., (1958 J. Chem. Soc. 4001) and Suvorov *et al*., (1962 J. Gen. Chem. USSR 31, 3409). Alternatively, the hydroxylation at the carbon-21 position of the precursor may comprise the steps of sulfonyloxaziridine oxygenation. Suitable techniques for accomplishing this step are described, for example, by Davis & Sheppard (1989 Tetrahedron 45, 5703) and by Elmore & Paquette (1993 J. Org. Chem. 58, 4693).

Preferably however, hydroxylation at the carbon-21 position may be performed by an enzyme system comprising hydroxylase enzyme(s) which effects hydroxylation at the carbon-21 position. As described above, such a system may comprise the enzyme(s) as a purified enzyme preparation or a cell-free extract, or produced *in situ* by an organism (typically an immobilised micro-organism). An example of such an enzyme is that obtainable from the organism *Aspergillus niger* ATCC 9142, which organism may be used in an enzyme system such as that defined above. A system suitable for the 21-hydroxylation of progesterone has been described by Holland & Auret (1975 Can. J. Chem. 53, 845-854).

In one embodiment, the 11β-hydroxylation and Δ¹⁻² dehydrogenation of the precursor are performed substantially simultaneously by an enzyme system comprising hydroxylase enzyme(s) capable of effecting 11β-hydroxylation and dehydrogenase enzyme(s) capable of effecting Δ¹⁻² dehydrogenation of the precursor. The term "substantially simultaneously" is intended, in particular, to encompass embodiments in which both steps are effected by a single enzyme system. For example, an enzyme system comprising a mixed culture of micro-organisms (e.g. a first micro-organism comprising a hydroxylase enzyme(s) and a second micro-organism comprising a dehydrogenase enzyme(s)) would be considered as effecting both a hydroxylation step and a dehydrogenation step "substantially simultaneously"). Preferably, the system comprises a combination of a species of *Curvularia (e.g. C. lunata*) for the 11β-hydroxylation step and a species of *Nocardia (e.g. N. simplex)* for the Δ¹⁻² dehydrogenation step. The Δ¹⁻² dehydrogenation may also be performed by species of *Bacillus* (e.g. *B. sphaericus*) or *Septomyxa* (e.g. *S. affinis*). Conveniently the enzyme system comprises a mixed culture of the two or more organisms which produce the necessary enzymes - this arrangement offers particular economy and ease of processing, (although characterisation of suitable enzymes in the future may allow the exploitation of recombinant DNA techniques to produce a single micro-organism which produces all of the necessary enzymes).

In an alternative embodiment, the 11β-hydroxylation and Δ¹⁻² dehydrogenation of the precursor are performed sequentially by separate systems, with a first system comprising hydroxylase enzyme(s) capable of effecting 11β-hydroxylation and a second system comprising dehydrogenase enzyme(s) capable of effecting Δ¹⁻² dehydrogenation of the precursor. In one embodiment, all three steps are performed by means of microbial biotransformation.

According to another aspect of the present invention there is provided a use of 17α-(acetyloxy)-6-methyl-,(6α)-pregn-4-ene-3,20-dione, or the derivatives thereof, represented in general formula (II) shown in Figure 2, in the production of 11β- 17α, 21-trihydroxy-6α-methyl, pregn-1,4-diene-3,20-dione or the derivatives thereof represented in general formula (I) shown in Figure 1.

The invention will now be described by way of the following illustrative examples and with reference to the accompanying drawings. The working practises of the methods described below will be familiar to those skilled in the art, In the drawings:
Figure 1 shows the structural formula of compounds according to general formula (I);
Figure 2 shows the structural formula of compounds according to general formula (II);
Figures 3-9 show reaction schemes of use in performance of the present invention;
Figures 10a and 10b are schematic representations of bioreactor systems of use in performance of the present invention;
Figures 11 to 14 show reaction schemes of use in performance of the present invention;
Figure 15 shows the core ring structure of steroids; and
Figures 16a and 16b show the D ring structure of progestogen steroids.

### Examples

### EXAMPLE 1

1.1. Biotransformation of pregn-4-ene-3,20-dione, 17-(acetyloxy)-6-methyl-, (6α)- (II) to the 11 β-hydroxy- derivative (III) by *Curvularia lunata* NRRL 2380. The reaction scheme is shown in Figure 3.
   *Curvularia lunata* NRRL 2380 was grown from stock culture in glucose/cornsteep liquor containing 10.0g/l glucose and 7.5g/l cornsteep liquor powder in 250 ml Erlenmeyer flasks, with the pH having been adjusted to pH 4.85 prior to autoclaving the media and inoculation with the fungal stock culture. The fungus was allowed to grow at 25°C for approximately 72 hours with shaking at 150 rpm on an orbital shaker prior to addition of 250 mg/l of (II) (dissolved in 2.5% v/v ethanol) to the seed flask. The steroid precursor (II), acted as an inducer of fungal hydroxylase(s) until such time as each mycelial mass contained significant levels of the relevant hydroxylating enzyme(s), which was a period of approximately 96 hours for *C. lunata.*
   The fungal mycelial mass was harvested at the appropriate time, washed with 25mM phosphate buffer, pH 6.5, and then used to prepare an immobilised washed-cell suspension using one of the following prior art immobilisation methods:
   a. entrapment in calcium alginate gel beads
   b. entrapment in κ-carrageenan gel beads
   c. entrapment in polyvinyl alcohol gel beads

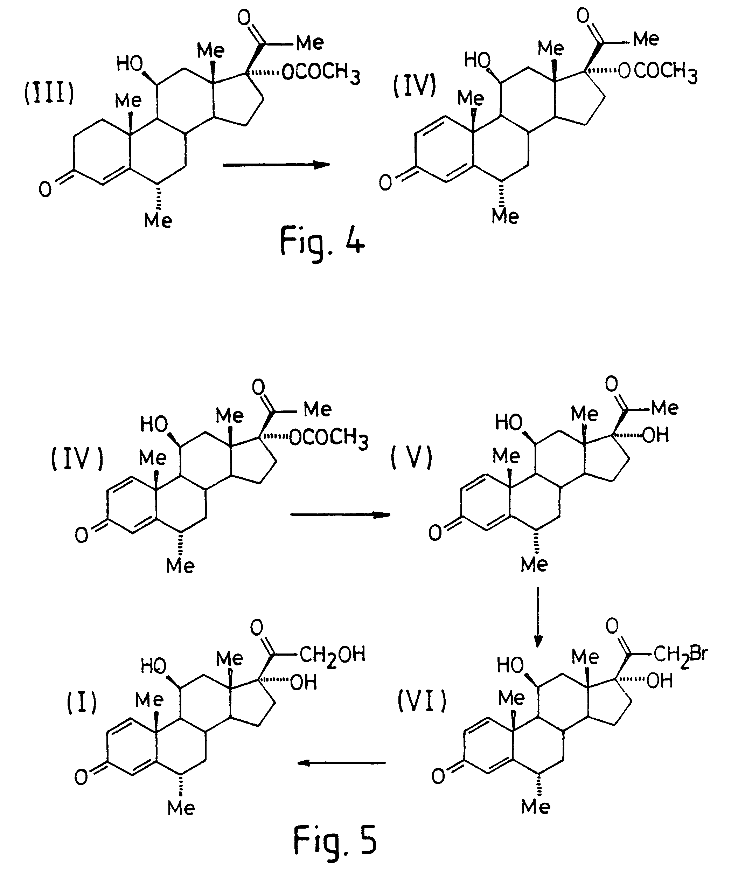

   The immobilised washed-cell preparation was placed in a suitable cylindrical container with flow-through facilities to thereby create a packed-bed bioreactor through which a dilute infusion of (II) was passed continuously by either upward or downward flow through the bioreactor. Samples from the bioreactor effluent stream were extracted and analysed for the 11 β-hydroxy derivative by Thin Layer Chromatography (TLC) and/or High Performance Liquid Chromatography (HPLC) methods known in the prior art.
   The samples to be tested were prepared as follows:- 0.5ml sample was added to 0.5ml ethylacetate to extract the steroid, 100 *µ*l of the ethylacetate layer was evaporated to dryness under nitrogen and redissolved in 20 *µ*l of ethanol. Around 1 *µ*l of the prepared sample was spotted on F254 silica plates for TLC, using a trichloromethane to ethanol (9:1) solvent and the TLC plate was developed under ultra violet light.
   For HPLC a reversed phase C18, ODS2, 20cm x 4.6mm column was used with a 70% methanol/water solvent eluting at 1 ml/min and detection at 242 nm.
1.2. Biotransformation of (III) from reaction 1.1 above to the Δ¹⁻² dehydrogenated derivative (IV) by *Nocardia simplex* ATCC 6946. The reaction scheme is shown in Figure 4.
   *Nocardia simplex* ATCC 6946 was grown at 30°C from stock culture in aliquots of yeast extract/peptone medium containing 250 mg/l of (III) as an inducer of microbial Δ¹⁻² dehydrogenase(s) until such time as each bacterial mass contained significant levels of the relevant dehydrogenating enzyme(s), which was a period of approximately 172 hours for *N. simplex.*
   The bacterial biomass was harvested at the appropriate time, washed with 25mM TES buffer, pH 6.5, and then used to prepare an immobilised washed-cell suspension using one of the following prior art immobilisation methods:
   a. entrapment in calcium alginate gel beads
   b. entrapment in *κ*-carrageenan gel beads
   c. entrapment in polyvinyl alcohol gel beads

   The immobilised washed-cell preparation was placed in a suitable cylindrical container with flow-through facilities to thereby create a packed-bed bioreactor through which a dilute infusion of the intermediate (III) from the bio-conversion in 1.1 above was passed continuously by either upward or downward flow through the bioreactor. Samples from the bioreactor effluent stream were extracted and analysed for the Δ¹⁻² dehydrogenated derivative (IV) by TLC and/or HPLC methods as described in 1.1.
1.3. Hydroxylation at the carbon-21 position of intermediate (IV) from reaction 1.2 above by chemical means to produce (I). The reaction scheme is shown in Figure 5.
   21-hydroxylation via a brominated intermediate was performed as described below.
   The 17α-acetoxy deprotection and bromination was carried out as follows:- to a stirring solution of (IV) in anhydrous tetrahydrofuran at 0°C, a solution of methanolic potassium hydroxide (10%w/v, 7.7eq) was added dropwise over 5 minutes at 0°C. The reaction mixture was allowed to warm to room temperature. After 4 hours the mixture was poured into water and diethyl ether, where upon the combined organic layers were washed with brine and water. The combined aqueous layers were further extracted with methylene chloride and ethyl ether. The combined organic layers were dried (MgSO₄) and the solvent removed *in vacuo* to afford a pale yellow crystalline solid. Recrystallisation from absolute ethanol gave the 'methyl ketone' hydrolysis product (V) as white granular crystals; 17α-hydroxy-6α-methylpregn-1,4-dien-3,20-dione.
   Bromination was conducted as follows:- a solution of (V) in anhydrous chloroform containing anhydrous ethanol was prepared at room temperature under nitrogen. The batch was cooled to -5°C (ice-salt bath), whereupon anhydrous hydrogen chloride was carefully hubbled (by means of sintered dispersion head) through the mixture with thorough stirring for 15-20 minutes. The colour of the solution changing from transparent colourless to transparent yellow. To the cooled batch a solution of bromine in anhydrous chloroform (6M, 1.1eq) was added dropwise with stirring over 10 minutes at 0°C. Upon completion of bromine addition the batch was allowed to warm to room temperature and stirred for a further 20 minutes, whereupon diethyl ether was added. The hatch was poured into water and the organic layer separated, washed with saturated sodium bicarbonate and water. The combined aqueous washings were further extracted with diethyl ether. The combined organic layers were dried (MgSO₄) and the solvent removed *in vacuo* to afford a pale yellow gum. Separation by flash chromatography eluting with ethyl acetate: light petroleum ether (1:2) afforded the '21-bromo-'adduct (VI); 21-bromo-17α-hydroxy-6α-methylpregn-1,4-dien-3,20-dione.
   Acetylation and deprotection to afford the 21-hydroxylated product was conducted as follows:- a solution of (VI) and anhydrous sodium acetate (2.81mmol, 2.6eq) in anhydrous *N,N*-dimethyl formamide was prepared at room temperature under nitrogen. After the suspension had been repeatedly evacuated and flushed with nitrogen (x5) the batch was heated to 60°C under nitrogen stream for 2 hours. On completion of the reaction time the batch was cooled to -18°C and ice water added. The mixture was stirred for a further 30 minutes under nitrogen at this temperature, whereupon the batch was poured into diethyl ether and water. The organic layer was separated and the aqueous layer extracted with diethyl ether. The combined organic layers were dried (MgSO₄) and the solvent removed *in vacuo* (plus, high vacuum overnight) to give the crude product as a pale yellow gum. Separation by flash chromatography eluting with ethyl acetate: light petroleum ether (1:2) gave the '21-acetate-' adduct; 21-acetoxy-17α-hydroxy-6α-methylpregn-1,4-dien-3,20-dione.
   Deprotection was achieved as follows: anhydrous potassium carbonate (2.5mmol, 10eq) was added to the '21-acetate-' adduct from above, in 95% aqueous ethanol at room temperature under nitrogen. The suspension was repeatedly evacuated and flushed with nitrogen (x5) and left stirring under nitrogen for 30 minutes. Water was added and the batch poured into diethyl ether. The aqueous layer was separated and extracted with diethyl ether. The combined organic layers were dried (MgSO₄) and the solvent removed *in vacuo* to afford a pale yellow crystalline solid. Separation by flash chromatography eluting with ethyl acetate: light petroleum ether (2:3) afforded (I).

### EXAMPLE 2

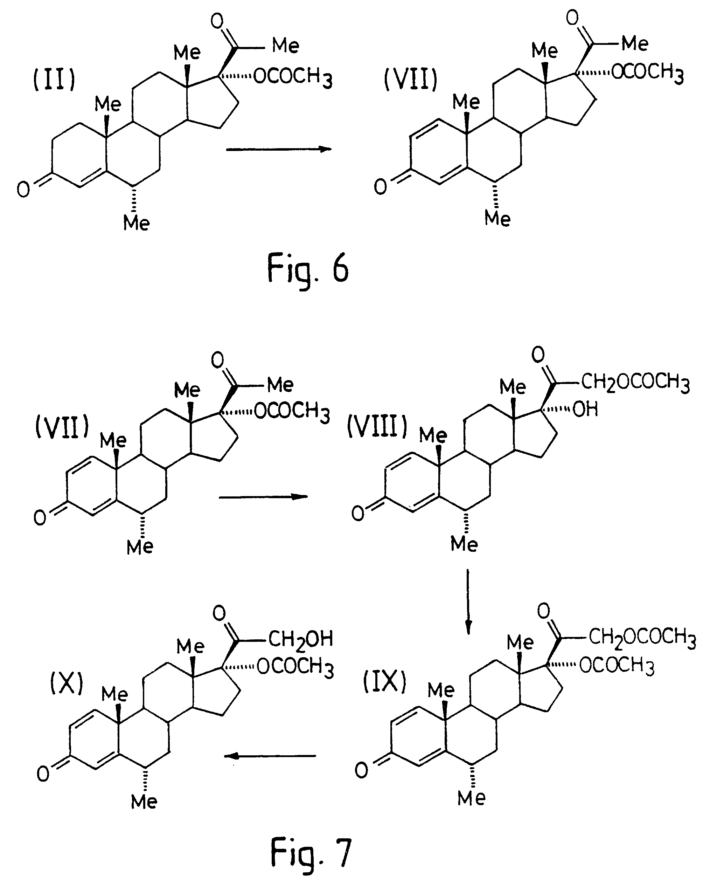

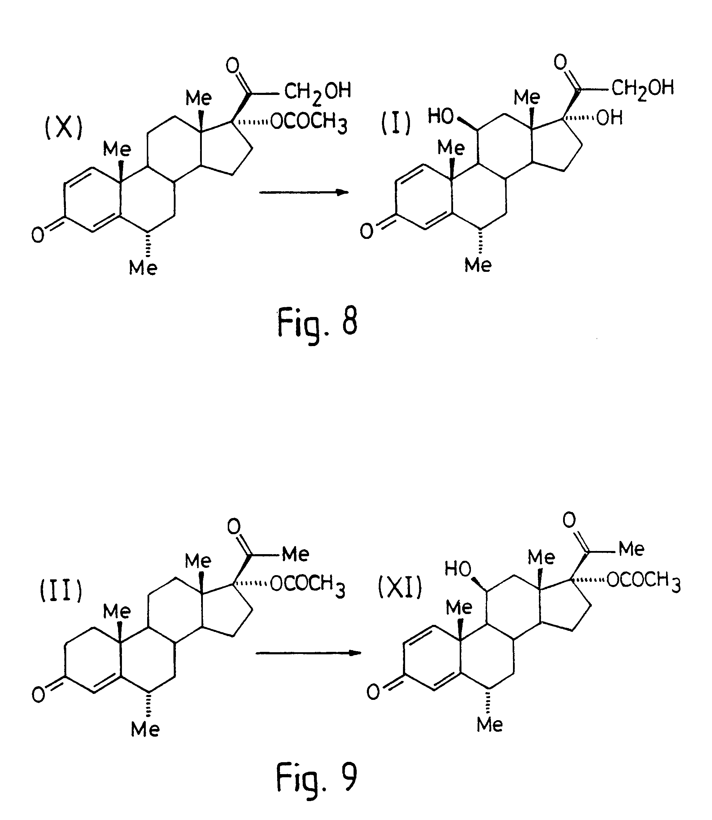

2.1. Δ¹⁻² dehydrogenation of (II) into (VII). The reaction scheme is shown in Figure 6.
   Traditionally selenium dioxide is used in this reaction. In an attempt to investigate more selective derivatives of this class of compound, benzeneseleninic anhydride and diphenyl diselenide were chosen as alternative selenium dioxide sources. We have shown that the Δ¹⁻² derivative of (II) was easily accessible using both stoichiometric selenium and catalytic selenium mediated dehydrogenations. Although reasonable yields were obtained using either procedure, the catalytic method uses less of the toxic selenium (about 90% less) than the non-catalytic procedures, and accordingly the catalytic method was preferred over the stoichiometric one for reasons of safety.
   In a round bottom flask, a stirring mixture of (II), iodosobenzoic acid (3.3eq) and diphenyl diselenide (0.2eq) in benzene was heated at reflux for 20 hours. The reaction mixture was cooled to room temperature, whereupon diethyl ether was added. The organic layer was separated and washed with a saturated solution of sodium bicarbonate (3x), water (2x), dried (MgSO₄) and the solvent removed *in vacuo* to afford a dark brown gum. Separation by flash chromatography, eluting with ethyl acetate and light petroleum ether (2:3), and recrystallisation from acetone/diethyl ether afforded the white crystalline dienone (VII); 17α-acetoxy-6α-methylpregn-1,4-diene-3,20-dione.
   Preparation of Iodosobenzoic acid; in a round bottom flask fitted with dropping addition funnel, acetic acid (11eq) was added dropwise to a vigorously stirred ice bath cooled (to below 40°C) suspension of *m*-iodobenzoic acid in sodium hypochlorite solution (∼14%w/v chlorine). Once all the acetic acid had been added, the yellow coloured reaction mixture was allowed to warm to room temperature. After the mixture had been stirring overnight, the reaction mixture was filtered under vacuum. The white precipitate was washed with water, acetone, diethyl ether and left to pull for 15 minutes. The white cake was dried under vacuum at room temperature overnight to give a fine white crystalline powder.
2.2. Hydroxylation at the Carbon-21 position of intermediate (VII) from reaction 2.1 to produce (X). The reaction scheme is shown in Figure 7.
   Hydroxylation at the carbon-21 position of (VII) using the Davis sulfonyloxaziridine oxygenation was accomplished upon treatment of (VII) with base in the presence of (±)-*trans* -2-(phenylsulfonyl)-3-phenyloxaziridine (previously prepared by treatment of *N* -benzylidenebenzenesulfonamide with Oxone® ). Oxone is a registered trade mark of E. I. duPont de Nemours, and refers to potassium peroxymonosulfate.
   A solution of (VII) in anhydrous tetrahydrofuran at -78°C under nitrogen was added dropwise via cannula to a stirring solution of potassium bis(trimethylsilylamide) (0.5M, 3.5eq) in anhydrous tetrahydrofuran at -78°C under nitrogen. The solution was stirred at this temperature for 30 minutes. In a separate vessel, a solution of (±)-*trans* -2-(phenylsulfonyl)-3-phenyloxaziridine (3.0eq) in anhydrous tetrahydrofuran at -78°C was also prepared under nitrogen and added dropwise via cannula to the substrate/base mixture. The reaction was stirred for a further 30 minutes at -78°C under nitrogen, whereupon the reaction mixture was quenched with a saturated solution of ammonium chloride. Without further isolation the crude mixture was acetylated with acetic anhydride (10eq) in freshly distilled pyridine (20eq) and methylene chloride (20eq) at room temperature overnight. Methanol was added, stirred for 45 minutes and solvent removed *in vacuo* (residual pyridine azeotroped with benzene) to afford (VIII); 21-acetoxy-17α-hydroxy-6α-methylpregn-1,4-diene-3,20-dione.
   Acetylation of the tertiary hydroxyl functionality at position 17 was effected by stirring a mixture of compound (VIII), acetic anhydride (36eq) and *para*-toluenesulfonic acid (1.7eq) in acetic acid at room temperature under nitrogen. After 4 hours, the mixture was diluted with water, extracted with diethyl ether and the ether layers washed with brine and water. The combined aqueous washings were re-extracted with methylene chloride and diethyl ether. The combined organic layers were dried (MgSO₄), solvent removed *in vacuo*, and after chromatography and recrystallisation, the diacetate intermediate (IX) isolated.
   Selective base hydrolysis of the 21-acetoxy functionality, without detriment to the 17α-acetoxyl group, was effected as follows:- to a stirring solution (IX) in anhydrous tetrahydrofuran at 0°C, a solution of methanolic potassium hydroxide (3% w/v) was added dropwise over 10 minutes at 0°C. After 2 hours at this temperature, the mixture was poured into water and diethyl ether. Separation was effected by flash chromatography eluting with ethyl acetate and light petroleum ether (2:1) and then by preparative TLC eluting with ethyl acetate and light petroleum ether (1:2) to afford the 17α-acetoxy-21-hydroxy intermediate (X).
   Preparation of (±)-*trans* -2-(Phenylsulfonyl)-3-phenyloxaziridine; to a vigorously stirring solution of *N* -benzylidenebenzenesulfonamide and potassium bicarbonate (8.3eq) in toluene in 500ml round bottom flask fitted with a dropping addition funnel, a solution of Oxone® (1.2eq) in water was added dropwise over 15 minutes. The reaction was stirred until all of the sulphonamide had been consumed. This was established by removing aliquots (∼1.0ml) from the mixture during the reaction, evaporating the solvent *in vacuo* and determining the ratio of sulphonamide (δ_{H} 8.7-9.2) to oxaziridine (5.4-6.0ppm) by nmr. After overnight stirring the aqueous layer was separated and extracted with toluene (2x). The combined organic layers were then washed with a 10% solution of sodium sulphite (2x), dried (MgSO₄) and the solvent removed *in vacuo* (ensuring the bath temperature did not exceed 40°C) to give, after trituration with a few mls of pentane, the pale yellow crystalline oxaziridine.
2.3. Bioconversion of intermediate (X) from reaction 2.2 into compound (I) using *Curvularia lunata* NRRL 2380. The reaction scheme is shown in Figure 8.
   *Curvularia lunata* NRRL 2380 was grown at 25°C from stock culture in glucose/cornsteep liquor containing 10.0g/l glucose and 7.5g/l cornsteep liquor powder in 250 ml Erlenmeyer flasks. with the pH having been adjusted to pH 4.85 prior to autoclaving the media and inoculation with the fungal stock culture. The fungus was allowed to grow for approximately 72 hours with shaking at 150 rpm on an orbital shaker prior to addition of 250 mg/l of (X) [dissolved in 2.5% v/v ethanol] to the seed flask. The intermediate (X) acted as an inducer of fungal hydroxylase(s) until such time as the mycelial mass was expected to contain significant levels of the relevant hydroxylating enzyme(s), which was a period of approximately 96 hours for *C. lunata* under the conditions described.
   The fungal mycelial mass was harvested at the appropriate time, washed with 25mM TES buffer, pH 6.5, and then used to prepare an immobilised washed-cell suspension using one of the following prior art immobilisation methods:
   a. entrapment in calcium alginate gel beads
   b. entrapment in κ-carrageenan gel beads
   c. entrapment in polyvinyl alcohol gel beads

   The immobilised washed-cell preparation was placed in a suitable cylindrical container with flow-through facilities to thereby create a packed-bed bioreactor through which a dilute infusion of the steroid precursor (X) was passed continuously by either upward or downward flow through the bioreactor. Samples of the bioreactor effluent stream were extracted and analysed for the 11 β-hydroxylated compound by TLC and/or HPLC methods, as described in example 1, paragraph 1.1 above.
   As a final step, deacetylation by base hydrolysis at the 17 position was performed as follows: to a stirring solution of the above reaction mixture in anhydrous tetrahydrofuran at 0°C, a solution of methanolic potassium hydroxide (10%w/v) was added dropwise over 5 minutes at 0°C. The reaction mixture was allowed to warm to room temperature. After 4 hours the mixture was poured into water and diethyl ether where the organic layers were washed with brine and water. The combined aqueous layers were further extracted with methylene chloride and diethyl ether. The combined organic layers were dried (MgSO₄) and the solvent removed *in vacuo* to afford, after chromatography and recrystallation, the hydrolysis product (I); 11β, 17α, 21-trihydroxy-6α-methylpregn-1,4-diene-3,20-dione.

### EXAMPLE 3

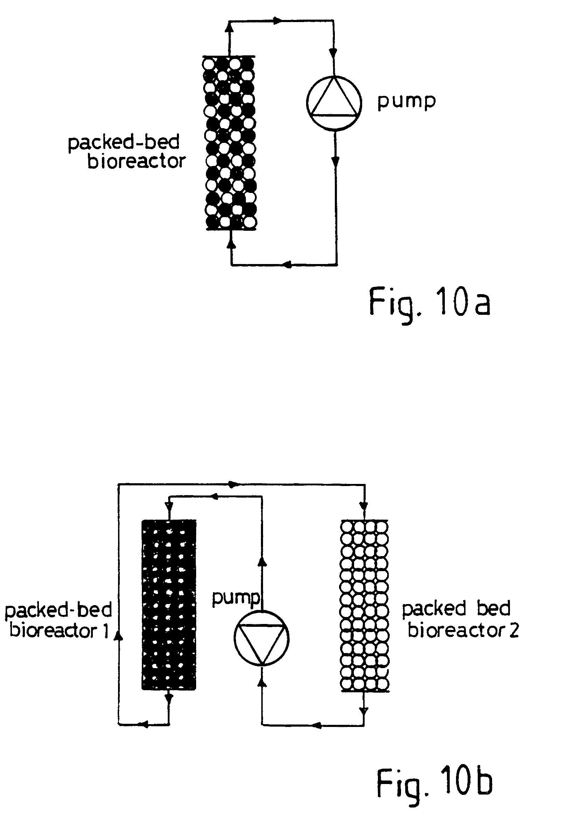

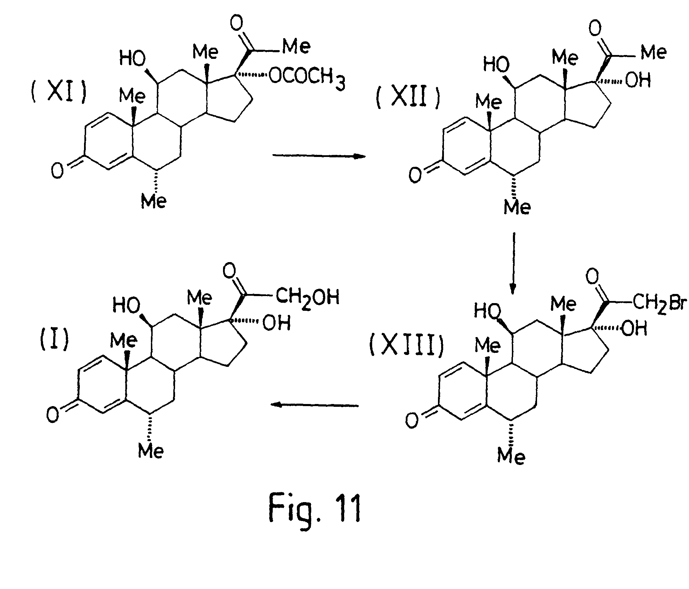

3.1. Biotransformation of the precursor (II) into the 11β-hydroxy, Δ¹⁻² dehydrogenated intermediate (XI) by a mixed culture of *C. lunata* NRRL 2380 and *Norcardia simplex* ATCC 6946. The reaction scheme is shown in Figure 9.
   *C. lunata* and *N. simplex* were first grown separately from stock cultures as described above and then reinoculated into aliquots of either fresh glucose/cornsteep liquor medium containing 250 mg/l of compound (II) or 25mM phosphate buffer pH 7.0 containing 250 mg/l (II) [dissolved in 2.5% ethanol] added to the medium to allow the mixed culture combination to biotransform the precursor to the 11 β-hydroxy-Δ¹⁻² dehydrogenated intermediate (XI) by consecutive and/or concurrent 11β-hydroxylation and Δ¹⁻² dehydrogenation. As part of a scale-up procedure the mixed culture was immobilised in packed-bed bioreactors (shown in Figures 10a and 10b).
   System 1 comprised single packed-bed bioreactor (Figure 10a) containing separate immobilised preparations of both an 11β-hydroxylating microorganism (•) and a Δ¹⁻² dehydrogenating microorganism (○).
   System 2 comprised two packed-bed bioreactors run in sequence (Figure 10b), one containing an immobilised preparation of an 11β-hydroxylating microorganism (•) and the other an immobilised preparation of a Δ¹⁻² dehydrogenating microorganism (○). It will be appreciated that the two bioreactors can be operated in any order.
   Both systems are shown as operating upward-flow pumping, but this could also be done by equivalent downward-flow circuits.
   The progress of each mixed culture biotransformation was followed by taking samples from the co-cultures at regular intervals, in each case the samples were extracted and analysed for steroids by HPLC as described in the previous examples.
3.2 Hydroxylation at the Carbon-21 position of the intermediate (XI) produced from the double biotransformation in 3.1 above. The reaction scheme is shown in Figure 11.
   21-hydroxylation via a brominated intermediate was performed as described below.
   The 17α-acetoxy deprotection and bromination was carried out as follows:- to a stirring solution of (XI) in anhydrous tetrahydrofuran at 0°C, a solution of methanolic potassium hydroxide (10%w/v, 7.7eq) was added dropwise over 5 minutes at 0°C. The reaction mixture was allowed to warm to room temperature. After 4 hours the mixture was poured into water and diethyl ether, where upon the combined organic layers were washed with brine and water. The combined aqueous layers were further extracted with methylene chloride and ethyl ether. The combined organic layers were dried (MgSO₄) and the solvent removed *in vacuo* to afford a pale yellow crystalline solid. Recrystallisation from absolute ethanol gave the 'methyl ketone' hydrolysis product (XII) as white granular crystals; 17a-hydroxy-6α-methylpregn-1,4-dien-3,20-dione.
   Bromination was conducted as follows:- a solution of (XII) in anhydrous chloroform containing anhydrous ethanol was prepared at room temperature under nitrogen. The batch was cooled to -5°C (ice-salt bath), whereupon anhydrous hydrogen chloride was carefully bubbled (by means of sintered dispersion head) through the mixture with thorough stirring for 15-20 minutes. The colour of the solution changing from transparent colourless to transparent yellow. To the cooled batch a solution of bromine in anhydrous chloroform (6M, 1.1eq) was added dropwise with stirring over 10 minutes at 0°C. Upon completion of bromine addition the batch was allowed to warm to room temperature and stirred for a further 20 minutes, whereupon diethyl ether was added. The batch was poured into water and the organic layer separated, washed with saturated sodium bicarbonate and water. The combined aqueous washings were further extracted with diethyl ether. The combined organic layers were dried (MgSO₄) and the solvent removed *in vacuo* to afford a pale yellow gum. Separation by flash chromatography eluting with ethyl acetate: light petroleum ether (1:2) afforded the '21-bromo-'adduct (XIII); 21-bromo-17α-hydroxy-6α-methylpregn-1,4-dien-3,20-dione.
   Acetylation and deprotection to afford the 21-hydroxylated product was conducted as follows:- a solution of (XIII) and anhydrous sodium acetate (2.81mmol, 2.6eq) in anhydrous *N,N*-dimethyl formamide was prepared at room temperature under nitrogen. After the suspension had been repeatedly evacuated and flushed with nitrogen (x5) the hatch was heated to 60°C under nitrogen stream for 2 hours. On completion of the reaction time the batch was cooled to -18°C and ice water added. The mixture was stirred for a further 30 minutes under nitrogen at this temperature, whereupon the batch was poured into diethyl ether and water. The organic layer was separated and the aqueous layer extracted with diethyl ether. The combined organic layers were dried (MgSO₄) and the solvent removed *in vacuo* (plus, high vacuum overnight) to give the crude product as a pale yellow gum. Separation by flash chromatography eluting with ethyl acetate: light petroleum ether (1:2) gave the '21-acetate-' adduct; 21-acetoxy-17α-hydroxy-6α-methylpregn-1,4-dien-3,20-dione.
   Deprotection was achieved as follows: anhydrous potassium carbonate (2.5mmol, 10eq) was added to the '21-acetate-' adduct from above, in 95% aqueous ethanol at room temperature under nitrogen. The suspension was repeatedly evacuated and flushed with nitrogen (x5) and left stirring under nitrogen for 30 minutes. Water was added and the batch poured into diethyl ether. The aqueous layer was separated and extracted with diethyl ether. The combined organic layers were dried (MgSO₄) and the solvent removed *in vacuo* to afford a pale yellow crystalline solid. Separation by flash chromatography eluting with ethyl acetate: light petroleum ether (2:3) afforded (I).

### EXAMPLE 4

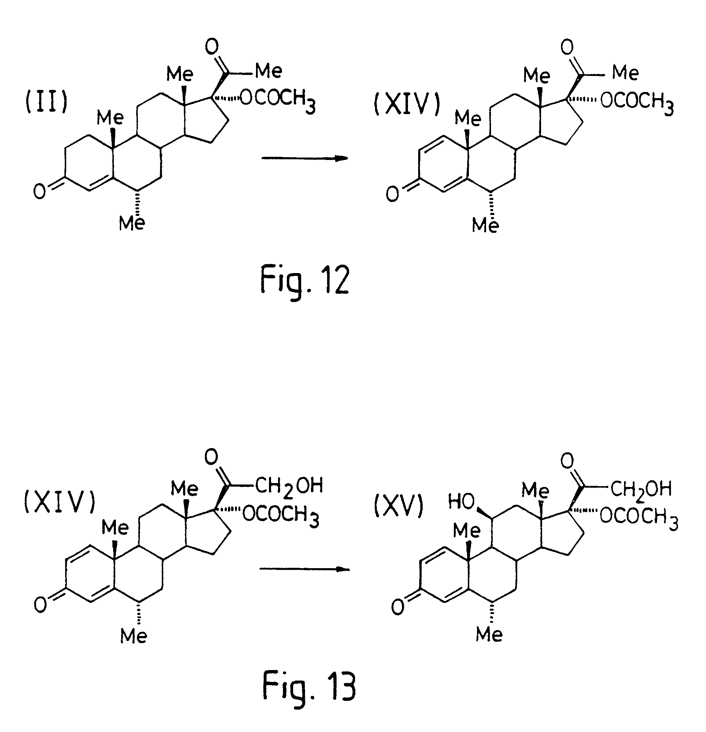

4.1 Selenium mediated Δ¹⁻²-dehydrogenation of (II) into (XIV). The reaction scheme is shown in Figure 12.
   In a round bottom flask, a stirring mixture of (II), iodosobenzoic acid (3.3eq, prepared as described in Example 2.1 above) and diphenyl diselenide (0.2eq) in benzene was heated at reflux for 20 hours. The reaction mixture was cooled to room temperature, whereupon diethyl ether was added. The organic layer was separated and washed with a saturated solution of sodium bicarbonate (3x), water (2x), dried (MgSO₄) and the solvent removed *in vacuo* to afford a dark brown gum. Separation by flash chromatography, eluting with ethyl acetate and light petroleum ether (2:3), and recrystallisation from acetone/diethyl ether afforded the white crystalline 'dienone' (XIV); 17α-acetoxy-6α-methylpregn-1,4-diene-3,20-dione.
4.2. Bioconversion of intermediate (XIV) from reaction 4.1 into compound (XV) using *Curvularia lunata* NRRL 2380. The reaction scheme is shown in Figure 13. *Curvularia lunata* NRRL 2380 was grown from stock culture as described in Example 2.3 above, prior to addition of 250 mg/l of (XIV) dissolved in 2.5% (v/v) ethanol to the seed flask. The intermediate (XIV) acted as an inducer of fungal hydroxylase(s) until such time as the mycelial mass was expected to contain significant levels of the relevant hydroxylating enzyme(s), which was a period of approximately 96 hours for *C. lunata*.
   The fungal mycelial mass was harvested at the appropriate time, washed with 25mM TES buffer, pH 6.5, and then used to prepare an immobilised washed-cell suspension using one of the following prior art immobilisation methods:
   a. entrapment in calcium alginate gel beads
   b. entrapment in κ-carrageenan gel beads
   c. entrapment in polyvinyl alcohol gel beads

   The immobilised washed-cell preparation was placed in a suitable cylindrical container with flow-through facilities thereby to create a packed-bed bioreactor through which a dilute infusion of the steroid precursor (XIV) was passed continuously by either upward or downward flow through the bioreactor. Samples of the bioreactor effluent stream were extracted and analysed for the 11 β-hydroxylated compound (XV) by TLC and/or HPLC methods as described in 1.1.
4.3. Hydroxylation at the Carbon-21 position of intermediate (XV) from reaction 4.2 to produce (I). The reaction scheme is shown in Figure 14.
   21-hydroxylation via a brominated intermediate was performed as follows:
   The 17α-acetoxy deprotection and bromination was carried out as follows:- to a stirring solution of (XV) in anhydrous tetrahydrofuran at 0°C, a solution of methanolic potassium hydroxide (10%w/v, 7.7eq) was added dropwise over 5 minutes at 0°C. The reaction mixture was allowed to warm to room temperature. After 4 hours the mixture was poured into water and diethyl ether, where upon the combined organic layers were washed with brine and water. The combined aqueous layers were further extracted with methylene chloride and ethyl ether. The combined organic layers were dried (MgSO₄) and the solvent removed *in vacuo* to afford a pale yellow crystalline solid. Recrystallisation from absolute ethanol gave the 'methyl ketone' hydrolysis product (XVI) as white granular crystals; 17α-hydroxy-6α-methylpregn-1,4-diene-3,20-dione.

Bromination was conducted as follows:- a solution of (XVI) in anhydrous chloroform containing anhydrous ethanol was prepared at room temperature under nitrogen. The batch was cooled to -5°C (ice-salt bath), whereupon anhydrous hydrogen chloride was carefully bubbled (by means of sintered dispersion head) through the mixture with thorough stirring for 15-20 minutes, the solution changing from a transparent colourless liquid to a transparent yellow liquid. To the cooled batch a solution of bromine in anhydrous chloroform (6M, 1.1eq) was added dropwise with stirring over 10 minutes at 0°C. Upon completion of bromine addition the batch was allowed to warm to room temperature and stirred for a further 20 minutes, whereupon diethyl ether was added. The batch was poured into water and the organic layer separated, washed with saturated sodium bicarbonate and water. The combined aqueous washings were further extracted with diethyl ether. The combined organic layers were dried (MgSO₄) and the solvent removed *in vacuo* to afford a pale yellow gum. Separation by flash chromatography eluting with ethyl acetate: light petroleum ether (1:2) afforded the '21-bromo-'adduct (XVII); 21-bromo-17α-hydroxy-6a-methylpregn-1,4-diene-3,20-dione.

Acetylation and deprotection to afford the 21-hydroxylated product was conducted as follows:- a solution of (XVII) and anhydrous sodium acetate (2.81mmol. 2.6eq) in anhydrous *N,N* -dimethyl formamide was prepared at room temperature under nitrogen. After the suspension had been repeatedly evacuated and flushed with nitrogen (x5) the batch was heated to 60°C under nitrogen stream for 2 hours. On completion of the reaction time the batch was cooled to -18°C and ice water added. The mixture was stirred for a further 30 minutes under nitrogen at this temperature, whereupon the batch was poured into diethyl ether and water. The organic layer was separated and the aqueous layer extracted with diethyl ether. The combined organic layers were dried (MgSO₄) and the solvent removed *in vacuo* (plus, high vacuum overnight) to give the crude product as a pale yellow gum. Separation by flash chromatography eluting with ethyl acetate: light petroleum ether (1:2) gave the '21-acetate-' adduct: 21-acetoxy-17α-hydroxy-6α-methylpregn-1,4-diene-3,20-dione.

Deprotection was achieved as follows: anhydrous potassium carbonate (2.5mmol, 10eq) was added to a solution of the '21-acetate-' adduct in 95% aqueous ethanol at room temperature under nitrogen. The suspension was repeatedly evacuated and flushed with nitrogen (x5) and left stirring under nitrogen for 30 minutes. Water was added and the batch poured into diethyl ether. The aqueous layer was separated and extracted with diethyl ether. The combined organic layers were dried (MgSO₄) and the solvent removed *in vacuo* to afford a pale yellow crystalline solid. Separation by flash chromatography eluting with ethyl acetate: light petroleum ether (2:3) afforded (I), completing the synthesis.

It will be appreciated that in the present invention the Δ¹⁻² dehydrogenation, 11β-hydroxylation and carbon-21 hydroxylation steps in the conversion of medroxyprogesterone acetate or its derivatives (II) into methylprednisolone or its derivatives (I) may be performed in any order and is not limited to the sequence of steps disclosed in the above examples.

It will further be appreciated that the 11β-hydroxylation step of the precursor molecule or its derivatives in the present invention may be performed by species of *Cunninghamella*, such as *C*. *elegans* NRRL 1369, or any other organism or system comprising the hydroxylase enzyme(s) to effect the 11β-hydroxylation of steroids. Furthermore the Δ¹⁻² dehydrogenation step of the precursor molecule or its intermediates in the present invention may be performed by species of *Bacillus sphaericus* ATCC 13805, *Rhodococcus equi* ATCC 14887, *Septomyxa affinis* ATCC 6737 or any other organism or system comprising the dehydrogenating enzyme(s) to effect the Δ¹⁻² dehydrogenation of steroids.
It will be further appreciated that the mixed culture biotransformation given in Example 3 may be any combination of organism(s) or system(s) capable of performing the 11β-hydroxylation and the Δ¹⁻² dehydrogenation of the steroid precursor molecule or its derivatives

## Claims

1. A method for the production of an adrenocorticoid steroid represented by the general formula (I) below: 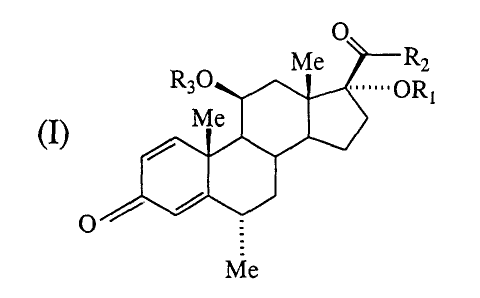 wherein R₁ may be H, alkyl, substituted alkyl, silyl, acyl, acyloxy or carbamoyl, R₂ may be methyl, mono- or disubstituted methyl, substituted alkyl, methyl or substituted alkylhydroxy, silyloxy, alkyloxy, acyl, acyloxy or carbamoyl, and R₃ is H; the method comprising treatment of a precursor thereof having the general formula (II) below: 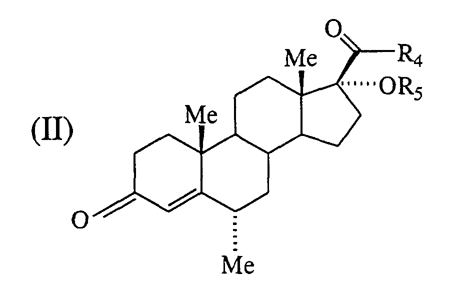 wherein R₄ may be methyl, mono- or disubstituted methyl, substituted alkyl, methyl or substituted alkyl hydroxy, silyloxy, alkyloxy, acyl, acyloxy or carbamoyl; and R₅ may be H, alkyl, substituted alkyl, silyl, acyl, acyloxy or carbamoyl, so as to effect the following steps: hydroxylation at the 11β position; formation of a double bond between positions 1 and 2; and hydroxylation at position 21.

2. A method according to claim 1, wherein R₁ is H or COCH₃ and R₂ is CH₂OH.

3. A method according to claim 1 or 2, wherein R₄ is CH₃ and R₅ is H or COCH₃.

4. A method according to any one of the preceding claims, wherein at least one of the method steps is performed by use of an enzyme system, in which an enzyme may be present in substantially pure form, or as a substantially cell-free extract, or produced *in situ* by a micro-organism.

5. A method according to claim 4, wherein the enzyme is produced *in situ* by an immobilised micro-organism.

6. A method according to any one of the preceding claims, wherein the 11β hydroxylation step is effected by use of an enzyme obtainable from *Curvularia* sp. or *Cunninghamella* sp.

7. A method according to any one of the preceding claims, wherein the formation of a double bond between positions 1 and 2 comprises a Δ¹⁻² dehydrogenation step effected by use of an enzyme system obtainable from *Nocardia* sp., *Bacillus* sp., *Septomyxa* sp. or *Rhodococcus* sp.

8. A method according to any one of the preceding claims, comprising use of an enzyme system in which the 11β hydroxylation and Δ¹⁻² dehydrogenation steps are performed substantially simultaneously.

9. A method according to any one of claims 1-7, comprising use of an enzyme system in which the 11β hydroxylation and Δ¹⁻² dehydrogenation steps are performed sequentially (in either order).

10. A method according to any one of the preceding claims, comprising use of an enzyme system in which the 11β hydroxylation step is effected by an enzyme obtainable from *Curvularia* sp. and the Δ¹⁻² dehydrogenation step is effected by an enzyme obtainable from *Nocardia* sp.

11. A method according to any one of claims 1-6, wherein the Δ¹⁻² dehydrogenation step is effected by chemical means using diphenyl diselenide as a catalyst, or a selenium-containing compound as a stoichiometric reagent.

12. A method according to any one of the preceding claims, wherein hydroxylation at position 21 is effected by use of an enzyme system obtainable from *Aspergillus* sp.

13. A method according to any one of claims 1-11, wherein hydroxylation at position 21 is effected by a method comprising sulfonyloxaziridine oxygenation, or bromination followed by elimination and saponification.

14. Use of a compound according to general formula (II) below 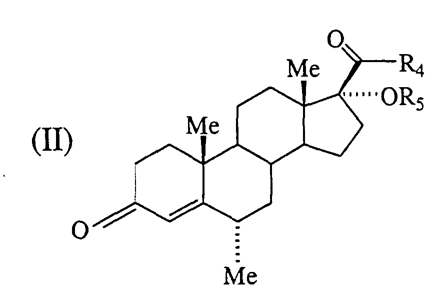 wherein R₄ may be methyl, mono- or disubstituted methyl, substituted alkyl, methyl or substituted alkyl hydroxy, silyloxy, alkyloxy, acyl, acyloxy or carbamoyl; and R₅ may be H, alkyl, substituted alkyl, silyl, acyl, acyloxy or carbamoyl,
as a starting material for the synthesis of an adrenocorticoid steroid of the general formula (I) below: 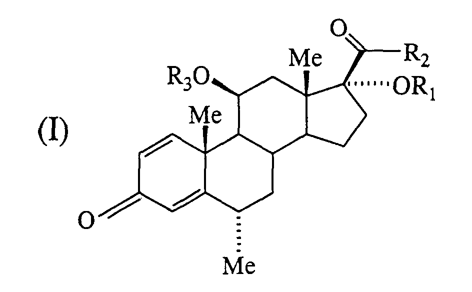 wherein R₁ may be H, alkyl, substituted alkyl, silyl, acyl, acyloxy or carbamoyl, R₂ may be methyl, mono- or disubstituted methyl, methyl or substituted alkylhydroxy, silyloxy, alkyloxy, acyl, acyloxy or carbamoyl, and R₃ is H; the method comprising treatment of a precursor thereof so as to effect the following steps: hydroxylation at the 11β position; formation of a double bond between positions 1 and 2; and hydroxylation at position 21.

15. Use of an intermediate compound in the production of an adrenocorticoid steroid according to general formula (I) below 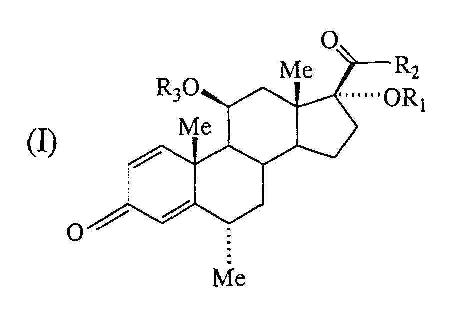 wherein R₁, may be H, alkyl, substituted alkyl, silyl, acyl, acyloxy or carbamoyl, R₂ may be methyl, mono- or disubstituted methyl, substituted alkyl, methyl or substituted alkylhydroxy, silyloxy, alkyloxy, acyl, acyloxy or carbamoyl, and R₃ is H; the method comprising treatment of a precursor thereof so as to effect the following steps: hydroxylation at the 11β position; formation of a double bond between positions 1 and 2; and hydroxylation at position 21, the intermediate compound being obtainable by performing on a precursor according to general formula (II) below any one or any two of the following steps : hydroxylation at the 11β position; formation of a double bond between positions 1 and 2; and hydroxylation at position 21: 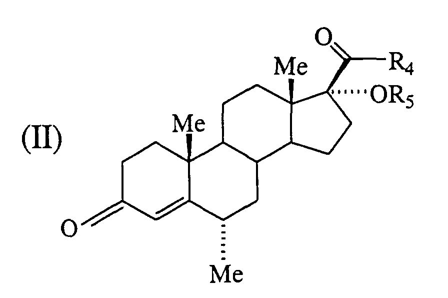 wherein R₄ may may be methyl, mono- or disubstituted methyl, substituted alkyl, methyl or substituted alkyl hydroxy, silyloxy, alkyloxy, acyl, acyloxy or carbamoyl; and R₅ may be H, alkyl, substituted alkyl, silyl, acyl, acyloxy or carbamoyl.

## Patentansprüche

1. Verfahren zur Herstellung eines durch die folgende allgemeine Formel (I) dargestellten Adrenocorticosteroids: 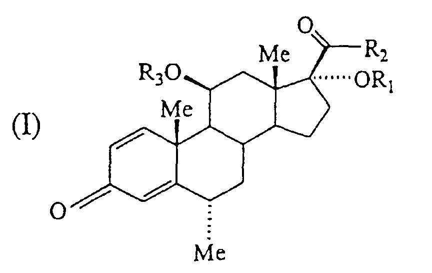 worin R₁ H, Alkyl, substituiertes Alkyl, Silyl, Acyl, Acyloxy oder Carbamoyl sein kann und R₂ Methyl, mono- oder disubstituiertes Methyl, substituiertes Alkyl, Methyl oder substituiertes Alkylhydroxy, Silyloxy, Alkyloxy, Acyl, Acyloxy oder Carbamoyl sein kann und R₃ H ist, wobei das Verfahren die Umsetzung einer die folgende allgemeine Formel (II) besitzenden Vorstufe davon umfaßt: 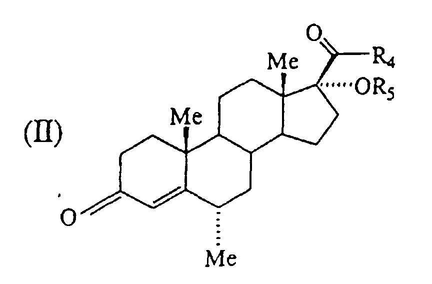 worin R₄ Methyl, mono- oder disubstituiertes Methyl, substituiertes Alkyl, Methyl oder substituiertes Alkylhydroxy, Silyloxy, Alkyloxy, Acyl, Acyloxy oder Carbamoyl sein kann und R₅ H, Alkyl, substituiertes Alkyl, Silyl, Acyl, Acyloxy oder Carbamoyl sein kann, um so die folgenden Schritte zu bewirken: Hydroxylierung an der 11β-Position; Bildung einer Doppelbindung zwischen Position 1 und 2; und Hydroxylierung an Position 21.

2. Verfahren gemäß Anspruch 1, wobei R₁ H oder COCH₃ und R₂ CH₂OH ist.

3. Verfahren gemäß Anspruch 1 oder 2, wobei R₄ CH₃ und R₅ H oder COCH₃ ist.

4. Verfahren gemäß einem der vorhergehenden Ansprüche, wobei wenigstens einer der Verfahrensschritte mit einem Enzymsystem durchgeführt wird, in dem ein Enzym in weitgehend reiner Form oder in einem weitgehend zellfreien Extrakt vorhanden sein kann oder von einem Mikroorganismus *in situ* hergestellt werden kann.

5. Verfahren gemäß Anspruch 4, wobei das Enzym von einem immobilisierten Mikroorganismus *in situ* hergestellt wird.

6. Verfahren gemäß einem der vorhergehenden Ansprüche, wobei der 11β-Hydroxylierungsschritt durch die Verwendung eines aus *Curvularia* sp. oder *Cunninghamella* sp. erhältlichen Enzyms bewirkt wird.

7. Verfahren gemäß einem der vorhergehenden Ansprüche, wobei die Bildung einer Doppelbindung zwischen Position 1 und 2 einen Δ¹⁻²-Dehydrierungsschritt umfaßt, der durch die Verwendung eines aus *Nocardia* sp., *Bacillus* sp., *Septomyxa* sp. oder *Rhodococcus* sp. erhältlichen Enzymsystems bewirkt wird.

8. Verfahren gemäß einem der vorhergehenden Ansprüche, welches die Verwendung eines Enzymsystems, in dem der 11β-Hydroxylierungs- und Δ¹⁻²-Dehydrierungsschritt weitgehend gleichzeitig durchgeführt werden, umfaßt.

9. Verfahren gemäß einem der Ansprüche 1-7, welches die Verwendung eines Enzymsystems, in dem der 11β-Hydroxylierungs- und Δ¹⁻²-Dehydrierungsschritt nacheinander (in beliebiger Reihenfolge) durchgeführt werden, umfaßt.

10. Verfahren gemäß einem der vorhergehenden Ansprüche, welches die Verwendung eines Enzymsystems, in dem der 11β-Hydroxylierungsschritt durch ein aus *Curvularia* sp. erhältliches Enzym und der Δ¹⁻²-Dehydrierungsschritt durch ein aus *Nocardia* sp. erhältliches Enzym bewirkt wird, umfaßt.

11. Verfahren gemäß einem der Ansprüche 1-6, wobei der Δ¹⁻²-Dehydrierungsschritt auf chemischem Wege durch die Verwendung von Diphenyldiselenid als Katalysator oder von einer Selen enthaltenden Verbindung als stöchiometrisches Reagens bewirkt wird.

12. Verfahren gemäß einem der vorhergehenden Ansprüche, wobei die Hydroxylierung an Position 21 durch die Verwendung eines aus *Aspergillus* sp. erhältlichen Enzymsystems bewirkt wird.

13. Verfahren gemäß einem der Ansprüche 1-11, wobei die Hydroxylierung an Position 21 durch ein Verfahren bewirkt wird, das eine Sulfonyloxaziridinoxidation oder eine Bromierung mit nachfolgender Eliminierung und Verseifung umfaßt.

14. Verwendung einer Verbindung gemäß der folgenden allgemeinen Formel (II) 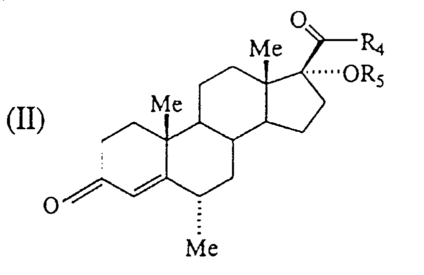 worin R₄ Methyl, mono- oder disubstituiertes Methyl, substituiertes Alkyl, Methyl oder substituiertes Alkylhydroxy, Silyloxy, Alkyloxy, Acyl, Acyloxy oder Carbamoyl sein kann und R₅ H, Alkyl, substituiertes Alkyl, Silyl, Acyl, Acyloxy oder Carbamoyl sein kann,
als Ausgangsmaterial für die Synthese eines Adrenocorticosteroids der folgenden allgemeinen Formel (I): 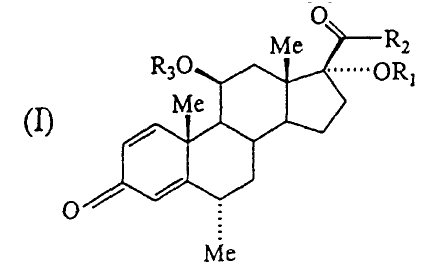 worin R₁ H, Alkyl, substituiertes Alkyl, Silyl, Acyl, Acyloxy oder Carbamoyl sein kann und R₂ Methyl, mono- oder disubstituiertes Methyl, Methyl oder substituiertes Alkylhydroxy, Silyloxy, Alkyloxy, Acyl, Acyloxy oder Carbamoyl sein kann und R₃ H ist, wobei das Verfahren die Umsetzung einer Vorstufe davon umfaßt, um so die folgenden Schritte zu bewirken: Hydroxylierung an der 11β-Position; Bildung einer Doppelbindung zwischen Position 1 und 2; und Hydroxylierung an Position 21.

15. Verwendung einer Zwischenverbindung in der Herstellung eines Adrenocorticosteroids gemäß der folgenden allgemeinen Formel (I) 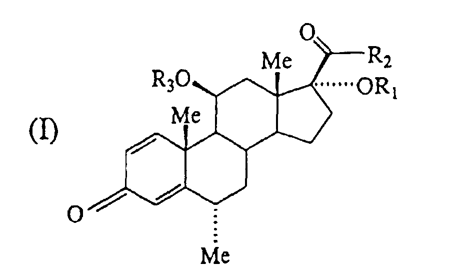 worin R₁ H, Alkyl, substituiertes Alkyl, Silyl, Acyl, Acyloxy oder Carbamoyl sein kann und R₂ Methyl, mono- oder disubstituiertes Methyl, substituiertes Alkyl, Methyl oder substituiertes Alkylhydroxy, Silyloxy, Alkyloxy, Acyl, Acyloxy oder Carbamoyl sein kann und R₃ H ist, wobei das Verfahren die Umsetzung einer Vorstufe davon umfaßt, um so die folgenden Schritte zu bewirken: Hydroxylierung an der 11β-Position; Bildung einer Doppelbindung zwischen Position 1 und 2; und Hydroxylierung an Position 21, wobei die Zwischenverbindung dadurch erhalten wird, daß man an der Vorstufe gemäß der folgenden allgemeinen Formel (II) einen oder zwei beliebige der folgenden Schritte durchführt: Hydroxylierung an der 11β-Position; Bildung einer Doppelbindung zwischen Position 1 und 2; und Hydroxylierung an Position 21: 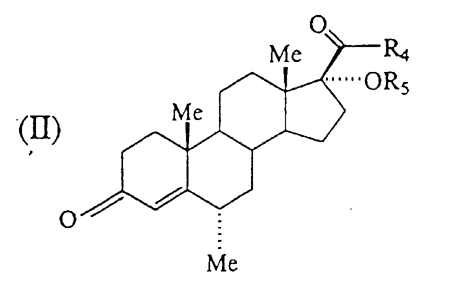 worin R₄ Methyl, mono- oder disubstituiertes Methyl, substituiertes Alkyl, Methyl oder substituiertes Alkylhydroxy, Silyloxy, Alkyloxy, Acyl, Acyloxy oder Carbamoyl sein kann und R₅ H, Alkyl, substituiertes Alkyl, Silyl, Acyl, Acyloxy oder Carbamoyl sein kann.

## Revendications

1. Procédé de production d'un adrénocorticostéroïde représenté par la formule générale ci-dessous (I): 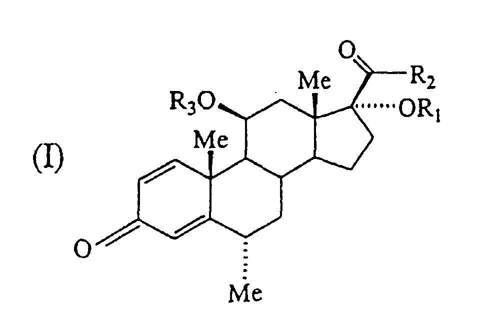 dans laquelle R₁ peut être H, alkyle, alkyle substitué, silyle, acyle, acyloxy ou carbamoyle, R₂ peut être méthyle, méthyl mono- ou disubstitué, alkyle substitué, méthyle ou alkylhydroxy substitué, silyloxy, alkyloxy, acyle, acyloxy ou carbamoyle, et R₃ est H; le procédé comprenant le traitement d'un précurseur de celui-ci ayant la formule générale ci-dessous (II): 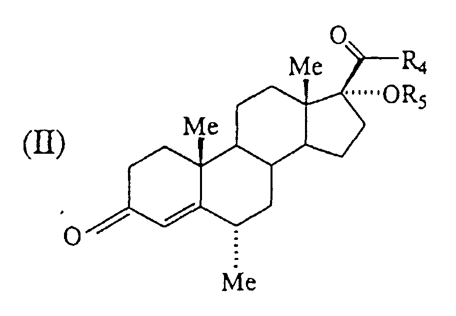 dans laquelle R₄ peut être méthyle, méthyl mono-ou disubstitué, alkyle substitué, méthyle ou alkylhydroxy substitué, silyloxy, alkyloxy, acyle, acyloxy ou carbamoyle; et R₅ peut être H, alkyle, alkyle substitué, silyle, acyle, acyloxy ou carbamoyle, pour réaliser les étapes suivantes: hydroxylation en position 11β; formation d'une double liaison entre les positions 1 et 2; et hydroxylation en position 21.

2. Procédé selon la revendication 1, **caractérisé en ce que** R₁ est H ou COCH₃ et R₂ est CH₂OH.

3. Procédé selon la revendication 1 ou 2, **caractérisé en ce que** R₄ est CH₃ et R₅ est H ou COCH₃.

4. Procédé selon l'une quelconque des revendications précédentes, **caractérisé en ce qu'**au moins l'une des étapes du procédé est réalisée en utilisant un système enzymatique, dans lequel une enzyme peut être présente sous forme sensiblement pure, ou sous forme d'extrait sensiblement dépourvu de cellules, ou produite *in situ* par un micro-organisme.

5. Procédé selon la revendication 4, **caractérisé en ce que** l'enzyme est produite *in situ* par un micro-organisme immobilisé.

6. Procédé selon l'une quelconque des revendications précédentes, **caractérisé en ce que** l'étape d'hydroxylation en 11β est réalisée en utilisant une enzyme susceptible d'être obtenue à partir de *Curvularia* sp. ou *Cunninghamella* sp.

7. Procédé selon l'une quelconque des revendications précédentes, **caractérisé en ce que** la formation d'une double liaison entre les positions 1 et 2 comprend une étape de déshydrogénation en Δ¹⁻² réalisée en utilisant un système enzymatique susceptible d'être obtenu à partir de *Nocardia* sp., *Bacillus* sp., *Septomyxa* sp. ou *Rhodococcus* sp.

8. Procédé selon l'une quelconque des revendications précédentes, comprenant l'utilisation d'un système enzymatique dans lequel les étapes d'hydroxylation en 11β et de déshydrogénation en Δ¹⁻² sont réalisées sensiblement simultanément.

9. Procédé selon l'une quelconque des revendications 1-7, comprenant l'utilisation d'un système enzymatique dans lequel les étapes d'hydroxylation en 11β et de déshydrogénation en Δ¹⁻² sont réalisées de façon séquentielle (dans n'importe quel ordre).

10. Procédé selon l'une quelconque des revendications précédentes, comprenant l'utilisation d'un système enzymatique dans lequel l'étape d'hydroxylation en 11β est réalisée par une enzyme susceptible d'être obtenue à partir de *Curvularia* sp. et l'étape de déshydrogénation en Δ¹⁻² est réalisée par une enzyme susceptible d'être obtenue à partir de *Nocardia* sp.

11. Procédé selon l'une quelconque des revendications 1-6, **caractérisé en ce que** l'étape de déshydrogénation en Δ¹⁻² est réalisée par un moyen chimique en utilisant le diséléniure de diphényle comme catalyseur, ou un composé sélénié comme réactif stoechiométrique.

12. Procédé selon l'une quelconque des revendications précédentes, **caractérisé en ce que** l'hydroxylation en position 21 est réalisée en utilisant un système enzymatique susceptible d'être obtenu à partir d'*Aspergillus* sp.

13. Procédé selon l'une quelconque des revendications 1-11, **caractérisé en ce que** l'hydroxylation en position 21 est réalisée par un procédé comprenant l'oxygénation par la sulfonyloxaziridine, ou la bromination suivie par l'élimination et la saponification.

14. Utilisation d'un composé selon la formule générale (II) ci-dessous 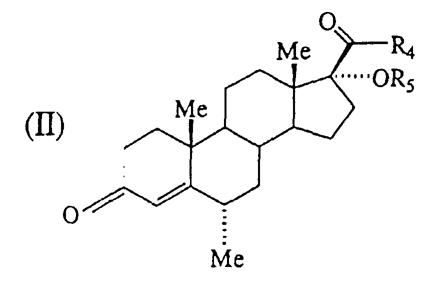 dans laquelle R₄ peut être méthyle, méthyl mono- ou disubstitué, alkyle substitué, méthyle ou alkylhydroxy substitué, silyloxy, alkyloxy, acyle, acyloxy ou carbamoyle; et R₅ peut être H, alkyle, alkyle substitué, silyle, acyle, acyloxy ou carbamoyle,
comme produit de départ pour la synthèse d'un adrénocorticostéroïde de formule générale (I) ci-dessous: 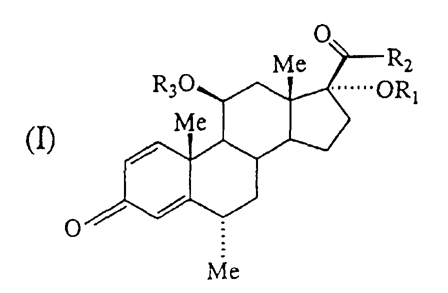 dans laquelle R₁ peut être H, alkyle, alkyle substitué, silyle, acyle, acyloxy ou carbamoyle, R₂ peut être méthyle, méthyl mono- ou disubstitué, méthyle ou alkylhydroxy substitué, silyloxy, alkyloxy, acyle, acyloxy ou carbamoyle, et R₃ est H; le procédé comprenant le traitement d'un précurseur de celui-ci de manière à réaliser les étapes suivantes: hydroxylation en position 11β; formation d'une double liaison entre les positions 1 et 2; et hydroxylation en position 21.

15. Utilisation d'un composé intermédiaire dans la production d'un adrénocorticostéroïde selon la formule générale (I) ci-dessous 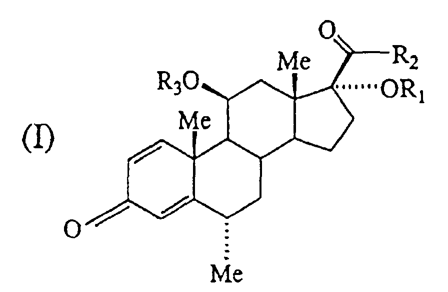 dans laquelle R₁ peut être H, alkyle, alkyle substitué, silyle, acyle, acyloxy ou carbamoyle, R₂ peut être méthyle, méthyl mono- ou disubstitué, alkyle substitué, méthyle ou alkylhydroxy substitué, silyloxy, alkyloxy, acyle, acyloxy ou carbamoyle, et R₃ est H; le procédé comprenant le traitement d'un précurseur de celui-ci de manière à réaliser les étapes suivantes: hydroxylation en position 11β; formation d'une double liaison entre les positions 1 et 2; et hydroxylation en position 21, le composé intermédiaire étant susceptible d'être obtenu en réalisant sur un précurseur selon la formule générale (II) ci-dessous l'une quelconque ou deux quelconques des étapes suivantes: hydroxylation en position 11β; formation d'une double liaison entre les positions 1 et 2; et hydroxylation en position 21: 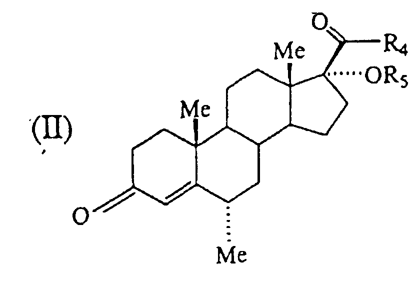 dans laquelle R₄ peut être méthyle, méthyl mono- ou disubstitué, alkyle substitué, méthyle ou alkylhydroxy substitué, silyloxy, alkyloxy, acyle, acyloxy ou carbamoyle; et R₅ peut être H, alkyle, alkyle substitué, silyle, acyle, acyloxy ou carbamoyle.
